# EUROPEAN PATENT APPLICATION

(11) **EP 4 538 371 A1**
(43) Date of publication of application: **16.04.2025**
(21) Application number: 23306793.3
(22) Date of filing: 13.10.2023
(51) Int. Cl.: C12N 9/99, C12Q 1/6895

(54) **STRAINS AND METHOD FOR REDUCING THE PRODUCTION OF HYDROGEN SULFIDE IN BEVERAGES**

(71) Applicant: Institut National de la Recherche pour l'Agriculture, l'Alimentation et l'Environnement, 75007 Paris (FR); Institut national d'enseignement supérieur pour l'agriculture, l'alimentation et l'environnement, 75116 Paris (FR)
(72) Inventor: LEGRAS, Jean-Luc, 34980 Saint Gely du Fesc (FR); BLONDIN, Bruno, 34070 Montpellier (FR); DE GUIDI, Irene, 34090 Montpellier (FR)
(74) Representative: J A Kemp LLP

(57) **Abstract**

The present invention relates to variants of the *YLR125W, ZRT2* and *SRN2* genes having the effect of indirectly modulating the production of hydrogen sulfide in *Saccharomyces,* and to the use of these variants for producing improved host cells and in methods for decreasing and/or reducing the production of hydrogen sulfide during alcoholic fermentation.

## Description

### BACKGROUND

Hydrogen sulfide (H₂S) is the main concern in the beverage industry since it gives a strong rotten egg aroma and has a low perception threshold (10 to 80 µg/L). In addition to its direct impact on wine odor H₂S can be the source of a set of compounds such as ethanethiol or dimethyl disulfide formed through chemical reactions in wines.

It has been reported that hydrogen sulfide was formed in situations of nutrient deficiencies while others have suggested that the production was rather independent of the grape must characteristics. Indeed, the strain's genetic background has been shown to play an important role in the capacity to release hydrogen sulfide. A huge variability has been described, both in terms of overall formation efficiency and timing of hydrogen sulfide release (Apostolos Spiropoulos et al. 2000). Indeed, variations in the sulfite reductase genes have been demonstrated to contribute to high sulfide formation (Kumar, Ramakrishnan, and Bisson 2010). The sulfite reductase has therefore been used as a target to reduce the capacity to release hydrogen sulfide during alcoholic fermentation. Mutated forms of *MET5* and *MET10* genes leading to enzymes with reduced activity have been shown to alter the formation of hydrogen sulfide in fermentation (Cordente et al. 2009). Application WO 2009/030863 and the publication by Marullo et al. (FEMS Yeast Res, 7, 1295-306, 2007) describe various markers associated with characteristics of interest in enological yeasts. One of these markers (*YOL083w*) located on chromosome XV is associated with a reduced hydrogen sulfide production. However, a draw-back of this strategy is that yeast tends to accumulate SO₂ that can't be efficiently reduced.

Using quantitative trait locus (QTL) approaches, Noble identified a variant of *MET2* encoding the homoserine O-trans-acetylase that decreased H₂S formation (Noble, Sanchez, and Blondin 2015). The contribution of a *MET2* variant on H₂S formation was also reported by Huang et al. (Huang, Roncoroni, and Gardner 2014). In the same study Noble pointed out the impact of *SKP2,* involved in the modulation of the sulfur reduction pathway, on H₂S release (Noble, Sanchez, and Blondin 2015). Variants of *SKP2* (*SKP2I350V* and *SKP2T357I*) displayed a reduced ability to release H₂S and SO2. *SKP2* encodes an F-box protein, which is predicted to be part of an *SCF* ubiquitin protease complex involved in the degradation of *Met14.* The allelic variants identified efficiently reduced H₂S and SO₂ excretion, likely because it targets *Met14* more efficiently and therefore enhances its degradation. Both gene variants *MET2* and *SKP2* have been used to breed wine *Saccharomyces cerevisiae* strains with reduced ability to form hydrogen sulfide.

Thus, there is a need in the art for improved strains and methods for reducing hydrogen sulfide levels in fermented beverages.

### BRIEF SUMMARY OF THE INVENTION

The present description provides compositions and methods for reducing hydrogen sulfide levels in fermented beverages.

The present description provides methods for decreasing and/or reducing hydrogen sulfide levels in a fermentation product or medium. In some embodiments, the methods comprise contacting the fermentation product or medium with a yeast strain, yeast cell or yeast culture comprising a polynucleotide encoding at least one of three genes contained in a region of 20kb on chromosome XII of *S. cerevisiae* strains.

The present description also provides a method for producing a fermentation product having a reduced hydrogen sulfide level, the method comprising fermenting a fermentation medium with a host cell comprising: a polynucleotide encoding a variant *YLR125W* polypeptide having at least about 70% identity to the amino acid sequence of SEQ ID NO: 2 and which comprises a substitution at position 34 (different from asparagine) and a C-terminal truncation; a polynucleotide encoding a variant *SRN2* polypeptide having at least about 70% identity to the amino acid sequence of SEQ ID NO: 4 and which comprises substitutions at position 133 (different from glycine) and at position 180 (different from asparagine); and/or a polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine), wherein the fermentation product has a reduced hydrogen sulfide level.

The present description further provides a fermentation product having a reduced hydrogen sulfide level comprising a host cell comprising: a polynucleotide encoding a variant *YLR125W* polypeptide having at least about 70% identity to the amino acid sequence of SEQ ID NO: 2 and which comprises a substitution at position 34 (different from asparagine) and a C-terminal truncation;a polynucleotide encoding a variant *SRN2* polypeptide having at least about 70% identity to the amino acid sequence of SEQ ID NO: 4 and which comprises substitutions at position 133 (different from glycine) and at position 180 (different from asparagine); and/or a polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine), wherein the fermentation product has a reduced hydrogen sulfide level.

The present description also provides a method of producing an improved host cell that produces reduced levels of hydrogen sulfide, the method comprising a step of: replacing an endogenous polynucleotide encoding a hydrogen sulfide producing *YLR125W* polypeptide with a polynucleotide encoding a variant *YLR125W* polypeptide having at least about 70% identity to the amino acid sequence of SEQ ID NO: 2 and which comprises a substitution at position 34 (different from asparagine) and a C-terminal truncation by introducing into a parent of the host cell said polynucleotide encoding the variant *YLR125W* polypeptide; replacing an endogenous polynucleotide encoding a hydrogen sulfide producing *SRN2* polypeptide with a polynucleotide encoding a variant *SRN2* polypeptide having at least about 70% identity to the amino acid sequence of SEQ ID NO: 4 and which comprises substitutions at position 133 (different from glycine) and at position 180 (different from asparagine) by introducing into a parent of the host cell said polynucleotide encoding the variant *SRN2* polypeptide; and/or replacing an endogenous polynucleotide encoding a hydrogen sulfide producing *ZRT2* polypeptide with a polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine) by introducing into a parent of the host cell said polynucleotide encoding the variant *ZRT2* polypeptide, wherein the parent of the improved host cell produces hydrogen sulfide

The present description also provides an improved host cell culture that produces reduced levels of hydrogen sulfide comprising a population of yeast cells, wherein the yeast cells comprise: an exogenous polynucleotide encoding a variant *YLR125W* polypeptide having at least about 70% identity to the amino acid sequence of SEQ ID NO: 2 and which comprises a substitution at position 34 (different from asparagine) and a C-terminal truncation; an exogenous polynucleotide encoding a variant *SRN2* polypeptide having at least about 70% identity to the amino acid sequence of SEQ ID NO: 4 and which comprises substitutions at position 133 (different from glycine) and at position 180 (different from asparagine); and/or an exogenous polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine), wherein the improved host cell culture produces reduced hydrogen sulfide levels in comparison to a culture of parent cells.

### BRIEF DESCRIPTION OF THE DRAWINGS

FIGURE 1 illustrates an overview of all genes present in the QTL_chrXII region on chromosome XII with genes of the present description in yellow impacting total hydrogen sulfide production.
FIGURE 2A illustrates the impact of variant change on the formation of hydrogen sulfide for the JN20 *YLR125W*JN27 and JN27 *YLR125W*JN20 strains and the corresponding parent JN20 and JN27 strains.
FIGURE 2B illustrates the impact of variant deletion on the formation of hydrogen sulfide for the JN20*ylr125w*Δ::natMX and JN27*ylr125w*Δ::natMX strains and the corresponding parent JN20 and JN27 strains.
FIGURE 3 illustrates the impact of variant change on the formation of hydrogen sulfide for the JN20*SRN*2JN27 and JN27*SRN*2JN20 strains and the corresponding parent JN20 and JN27 strains.
FIGURE 4 illustrates the impact of variant change on the formation of hydrogen sulfide for the JN20*ZRT*2JN27 and JN27*ZRT*2JN20 strains and the corresponding parent JN20 and JN27 strains.
FIGURE 5 illustrates the impact of a combination of the three variant forms of *YLR125W*JN27*, SRN2*JN27 and *ZRT2*JN27 in strain JN20 and the combination of the three variant forms of *YLR125W*JN20*, SRN2*JN20 and *ZRT*2JN20 in strain JN27.

### DETAILED DESCRIPTION OF THE INVENTION

The present description has now identified alleles of three genes indirectly involved in sulfur metabolism in *Saccharomyces,* as being associated with a reduced production of hydrogen sulfide.

It was unexpectedly discovered that hydrogen sulfide production can be impacted in a very indirect manner by various yeast metabolisms and cellular functions, underscoring their interactions between the sulfur assimilation pathway (SAP). Since the analysis was performed with yeast strains that are moderate, low, hydrogen sulfide producers, the data of the present description suggest that components directly involved in the SAP are likely responsible for high sulfide formation, while other cellular functions support only weaker productions.

One specific feature of the present description is the localization of three genes modulating hydrogen sulfide production in a single QTL. The identification of the QTL was likely facilitated by the clustering of these three genes, with alleles acting in the same direction in this region. From a technological point of view, this is a great advantage, because it means that the entire area has the potential to be introgressed in a desired strain followed by repeated backcrosses to improve it.

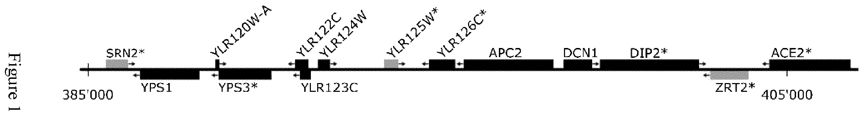

The three genes of the present description are located in the same QTL_chrXII region, within 20 kb regions on chromosome XII (Figure 1). The presence of several genes with opposite effects have been reported before in a single QTL (Ben-Ari et al. 2006; Brice et al. 2014), however it is to our knowledge the first time that three genes acting in a similar manner are found in a single small genomic region. Without being bound to any theory, this may suggest the presence of a putative cluster involved in sulfur or zinc metabolism.

### Definitions

A "must" as used herein refers to an unfermented mixture of fruit juice, stem fragments, fruit skins, seeds and/or pulp produced by mashing the fruit. Any fruits containing fermentable sugar such as, for example, grapes, apples, cherries, peaches, nectarines, plums, apricots, pears, persimmons, pineapples, mangoes, kiwis, strawberries, raspberries, blueberries, elderberries, blackberries, cranberries, figs, and loquats can be used. The fruits may be dried, boiled, poached, or otherwise processed prior to mashing. A must may comprise two or more fruits.

"Wort" as used herein refers to an unfermented liquid produced by mashing grains and/or grain hulls. Any grains containing fermentable sugar such as, for example, barley, wheat, rye, barley, rice, corn and oats can be used. The grains may be roasted, flaked, or otherwise processed prior to mashing. A wort may be produced from a mixture comprising two or more grains.

"Fermentation media" or "fermentation medium" as used herein refers to an unfermented mixture prior to addition of yeast. Fermentation media include, e.g., musts and worts. Fermentation media may further comprise an additional sugar source (e.g., honey, cane sugar, beet sugar, corn sugar, fructose, sucrose, or glucose); acid (e.g., citric acid, malic acid, tartaric acid, and mixtures thereof) and yeast nutrients (e.g., diammonium phosphate or another nitrogen source, vitamins, and the like).

As used herein, an "exogenous" polynucleotide sequence or amino acid sequence is introduced into a parent host cell or parent yeast strain by the action of man. The introduction into the host cell of the exogenous polynucleotide sequence or exogenous amino acid sequence can be by any means known in the art, including recombinant methods or classical yeast breeding methods (e.g., back-crossing).

The terms "polynucleotide" refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs). Unless otherwise indicated, a particular polynucleotide sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). The term nucleic acid is used interchangeably with gene, cDNA, mRNA, oligonucleotide, and polynucleotide.

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high-performance liquid chromatography. A protein that is the predominant species present in a preparation is substantially purified. The term "purified" denotes that a polynucleotide or protein gives rise to essentially one band in an electrophoretic gel. Particularly, it means that the nucleic acid or protein is at least 85% pure, more preferably at least 95% pure, and most preferably at least 99% pure.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, ÿ-carboxyglutamate, and O-phosphoserine. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

As used herein, a "hydrogen sulfide producing" polypeptide can produce hydrogen sulfide in the sulfur assimilation pathway (SAP). In the present description, a hydrogen sulfide producing *YLR125W* polypeptide will have an asparagine residue at position 34; a hydrogen sulfide producing *SRN2* polypeptide will have an asparagine residue at position 180 and a glycine residue at position 133; and/or a hydrogen sulfide producing *ZRT2* polypeptide will have glycine at position 19, a lysine residue at position 322 and a methionine residue at position 325.

As used herein, a "hydrogen sulfide reducing" variant of a polypeptide decreases and/or reduce hydrogen sulfide production in a very indirect manner by various yeast metabolisms and cellular functions, underscoring their interactions between the sulfur assimilation pathway (SAP). In the present description, a hydrogen sulfide reducing variant of the *YLR125W* polypeptide will comprises a substitution at position 34 that is different from asparagine and has a C-terminal truncation; a hydrogen sulfide reducing variant of the *SRN2* polypeptide will comprise a substitution at position 180 that is different from asparagine and at position 133 that is different from glycine; and/or a hydrogen sulfide reducing variant of the *ZRT2* polypeptide will comprises substitutions at position 19 that is different from glycine, at position 322 that is different from lysine, and at position 325 that is different from methionine.

The present disclosure provides host cells that produce decreased or reduced levels of hydrogen sulfide and express at least one of an exogenous polynucleotide encoding a variant of the *YLR125W* polypeptide; an exogenous polynucleotide encoding a variant of the *SRN2* polypeptide, or an exogenous polynucleotide encoding a variant of the *ZRT2* polypeptide as described herein.

In an embodiment, the exogenous polynucleotide encodes a variant of the *YLR125W* polypeptide having the amino acid sequence of SEQ ID NO: 2 and comprises a substitution at position 34 (different from asparagine) and a C-terminal truncation. In another embodiment, the polynucleotide encodes a variant of the *YLR125W* polypeptide having the amino acid sequence of SEQ ID NO: 2 producing reduced levels of hydrogen sulfide, and comprises a substitution at position 34 (different from asparagine) and a C-terminal truncation. In a further embodiment, the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 (different from asparagine) and a C-terminal truncation. In yet a further embodiment, the polynucleotide encodes a variant of the*YLR125W* polypeptide having the amino acid sequence of SEQ ID NO: 2 producing reduced levels of hydrogen sulfide, and comprises a substitution at position 34 that is lysine and a C-terminal truncation. In yet another embodiment, the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 that is lysine and . In yet another embodiment, the polynucleotide encoding the variant of the *YLR125W* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO:1.

In an embodiment, the exogenous polynucleotide encodes a variant of the *SRN2* polypeptide having the amino acid sequence of SEQ ID NO: 4 and comprises substitutions at positions 180 (different from asparagine) and at position 133 (different from glycine). In another embodiment, the polynucleotide encodes the variant of the *SRN2* polypeptide having the amino acid sequence of SEQ ID NO: 4 producing reduced levels of hydrogen sulfide, and comprises substitutions at position 180 (different from asparagine) and at position 133 (different from glycine). In a further embodiment, the variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and wherein the variant has the amino acid sequence of SEQ ID NO: 4 and comprises substitutions at position 180 (different from asparagine) and at position 133 (different from glycine). In yet a further embodiment, the polynucleotide encodes the variant of the *Saccharomyces SRN2* polypeptide having the amino acid sequence of SEQ ID NO: 4 producing reduced levels of hydrogen sulfide, and comprising substitutions at positions 133 and 180 that are respectively glutamic acid and aspartic acid. In yet another embodiment, the variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprising substitutions at position 133 and 180 that are respectively glutamic acid and aspartic acid. In an embodiment, the polynucleotide encoding the variant of the *SRN2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.

In an embodiment, the exogenous polynucleotide encodes a variant of the *ZRT2* polypeptide having the amino acid sequence of SEQ ID NO: 6 and comprises substitutions at position 19 (different from glycine), at position 322 (different from lysine), and at position 325 (different from methionine). In another embodiment, the polynucleotide encodes the variant of the *ZRT2* polypeptide having the amino acid sequence of SEQ ID NO: 6 producing reduced levels of hydrogen sulfide, and comprises substitutions at position 19 (different from glycine), at position 322 (different from lysine), and at position 325 (different from methionine). In a further embodiment, the variant of the *Saccharomyces ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position 19 (different from glycine), at position 322 (different from lysine), and at position 325 (different from methionine). In yet a further embodiment, the polynucleotide encodes the variant of the *ZRT2* polypeptide having the amino acid sequence of SEQ ID NO: 6 producing reduced levels of hydrogen sulfide, and comprises substitutions at positions19, 322 and 325 that are respectively aspartic acid, glutamic acid and threonine. In yet another embodiment, the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at positions19, 322 and 325 that are respectively aspartic acid, glutamic acid and threonine. In an embodiment the polynucleotide encoding a variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.

In another embodiment, host cells of the present description produce decreased and/or reduced levels of hydrogen sulfide and express at least two of the exogenous polynucleotides of the preceding embodiments. In yet another embodiment, host cells of the present description produce decreased or reduced levels of hydrogen sulfide and express the three exogenous polynucleotides of the preceding embodiments. In an embodiment, the host cells of the preceding embodiments may include one or more copies of the at least one of an exogenous polynucleotide of the preceding embodiment.

The present description also provides a method of producing an improved host cell that produces decreased and/or reduced levels of hydrogen sulfide. In an embodiment, the method comprises at least one step of: replacing an endogenous polynucleotide encoding a hydrogen sulfide producing *YLR125W* polypeptide with a polynucleotide encoding a hydrogen sulfide reducing variant of the *YLR125W* polypeptide by introducing into a parent of the yeast cell the polynucleotide encoding the hydrogen sulfide reducing variant of the *YLR125W* polypeptide, wherein the variant has the amino acid sequence of SEQ ID NO: 2 and comprises a substitution at position 34 (different from asparagine) and a C-terminal truncation; replacing an endogenous polynucleotide encoding a hydrogen sulfide producing *SRN2* polypeptide with a polynucleotide encoding a hydrogen sulfide reducing variant of the *SRN2* polypeptide by introducing into a parent of the yeast cell the polynucleotide encoding the hydrogen sulfide reducing variant of the *SRN2* polypeptide, wherein the variant has the amino acid sequence of SEQ ID NO: 4 and comprises substitutions at position 180 (different from asparagine) and at position 133 (different from glycine), or replacing an endogenous polynucleotide encoding a hydrogen sulfide producing *ZRT2* polypeptide with a polynucleotide encoding a hydrogen sulfide reducing variant of the *ZRT2* polypeptide by introducing into a parent of the yeast cell the polynucleotide encoding the hydrogen sulfide reducing variant of the *ZRT2* polypeptide, wherein the variant has the amino acid sequence of SEQ ID NO: 6 and comprises substitutions at position 19 (different from glycine), at position 322 (different from lysine), and at position 325 (different from methionine); wherein the parent of the improved host cell produces hydrogen sulfide.

In an embodiment, the polynucleotide encodes the hydrogen sulfide reducing variant of the *YLR125W* polypeptide having the amino acid sequence of SEQ ID NO: 2 producing reduced levels of hydrogen sulfide, and comprises a substitution at position 34 (different from asparagine) and a C-terminal truncation. In another embodiment, the hydrogen sulfide reducing variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid of SEQ ID NO: 2, and comprises a substitution at position 34 (different from asparagine) and a C-terminal truncation. In a further embodiment, the polynucleotide encodes the hydrogen sulfide reducing variant of the *YLR125W* polypeptide having the amino acid sequence of SEQ ID NO: 2 producing reduced levels of hydrogen sulfide, and comprising a substitution at position 34 that is lysine and a C-terminal truncation. In yet another embodiment, the hydrogen sulfide reducing variant of the*YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid of SEQ ID NO: 2, and comprises a substitution at position 34 that is lysine and a C-terminal truncation. In yet a further embodiment, the polynucleotide encoding the hydrogen sulfide reducing variant of the *YLR125W* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO:1.

In an embodiment, the polynucleotide encodes the hydrogen sulfide reducing variant of the *SRN2* polypeptide having the amino acid sequence of SEQ ID NO: 4 producing reduced levels of hydrogen sulfide, and comprises substitutions at position 180 (different from asparagine) and at position 133 (different from glycine). In another embodiment, the hydrogen sulfide reducing variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a *SRN2* of SEQ ID NO: 4, and comprises substitutions at position 180 (different from asparagine) and at position 133 (different from glycine). In a further embodiment, the polynucleotide encodes the hydrogen sulfide reducing variant of the *SRN2* polypeptide having the amino acid sequence of SEQ ID NO: 4 producing reduced levels of hydrogen sulfide, and comprises substitutions at position 133 and 180 that are respectively glutamic acid and aspartic acid. In yet another embodiment, the hydrogen sulfide reducing variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a *SRN2* of SEQ ID NO: 4, and comprises substitutions at positions 133 and 180 that are respectively glutamic acid and aspartic acid. In yet a further embodiment, the polynucleotide encoding the hydrogen sulfide reducing variant of the SRN2 polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.

In an embodiment, the polynucleotide encodes the hydrogen sulfide reducing variant of the *ZRT2* polypeptide having the amino acid sequence of SEQ ID NO: 6 producing reduced levels of hydrogen sulfide, and comprises substitutions at position 19 (different from glycine), at position 322 (different from lysine), and at position 325 (different from methionine). In another embodiment, the hydrogen sulfide reducing variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 6, and comprises substitutions at position 19 (different from glycine), at position 322 (different from lysine), and at position 325 (different from methionine). In yet another embodiment, the polynucleotide encodes the hydrogen sulfide reducing variant of the *ZRT2* polypeptide having the amino acid sequence of SEQ ID NO: 6 producing reduced levels of hydrogen sulfide, and comprises substitutions at position 19, 322 and 325 that are respectively aspartic acid, glutamic acid and threonine. In yet a further embodiment, the hydrogen sulfide reducing variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a *ZRT2* of SEQ ID NO: 6, and comprises substitutions at position 19, 322 and 325 that are respectively aspartic acid, glutamic acid and threonine. In an embodiment, the polynucleotide encoding a hydrogen sulfide reducing variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.

In an embodiment, host cells that produce low or decreased or reduced levels of hydrogen sulfide produce 50% or less hydrogen sulfide in comparison to the parent strain before introducing the at least one of an exogenous polynucleotide encoding a *YLR125W* polypeptide or an allele thereof; an exogenous polynucleotide encoding a *SRN2* polypeptide or an allele thereof, or an exogenous polynucleotide encoding a *ZRT2* polypeptide or a allele thereof as described herein. In some embodiments, host cells that produce low or decreased levels of hydrogen sulfide produce 40%, 30%, 25%, 20%, or less hydrogen sulfide in comparison to the parent strain before introducing the at least one of an exogenous polynucleotide encoding a *YLR125W* polypeptide or an allele thereof; an exogenous polynucleotide encoding a *SRN2* polypeptide or an allele thereof, or an exogenous polynucleotide encoding a *ZRT2* polypeptide or a allele thereof as described herein.

In a further embodiment of the method of producing an improved host cell, the method comprises replacing two endogenous polynucleotides encoding a hydrogen sulfide producing polypeptide of the preceding embodiments with the respective polynucleotides encoding a hydrogen sulfide reducing variant of the polypeptide of the preceding embodiments by introducing into a parent of the yeast cell the respective polynucleotide encoding the hydrogen sulfide reducing variant of the polypeptides of the preceding embodiments. In yet a further embodiment of the method of producing an improved host cell, the method comprises replacing the three endogenous polynucleotides encoding a hydrogen sulfide producing polypeptide of the preceding embodiments with the respective polynucleotides encoding a hydrogen sulfide reducing variant of the polypeptides of the preceding embodiments by introducing into a parent of the yeast cell the polynucleotides encoding the hydrogen sulfide reducing variants of the polypeptides of the preceding embodiments. In an embodiment of the method of producing an improved host cell, the method also includes introducing into a parent of the yeast cell one or more copies of the polynucleotides encoding the hydrogen sulfide reducing variants of the polypeptides of the preceding embodiments.

In an embodiment, the variant of the *YLR125W* polypeptide having the amino acid sequence of SEQ ID NO: 2 encompasses: a variant (more specifically known as *YLR125W34K* variant) encoding an *YLR125W* protein comprising a substitution at position 34 that is lysine and a C-terminal truncation.

In another embodiment, the variant of the *SRN2* polypeptide having the amino acid sequence of SEQ ID NO: 4 encompasses: a variant (more specifically known as *SRN2133E180X* variant) encoding *srn2* protein in which the amino acid at position 180 are different from aspartic acid, and the amino acid at position 133 is glutamic acid; a variant (more specifically known as *SRN2133X180D* variant) encoding *srn2* protein in which the amino acid at position 180 is aspartic acid and the amino acid at position 133 is different from glutamic acid; a variant (more specifically known as *SRN2133E180D* variant) encoding *srn2* protein in which the amino acid at position 180 is aspartic acid and the amino acid at position 133 is glutamic acid.

In yet an embodiment, the variant of the *ZRT2* polypeptide having the amino acid sequence of SEQ ID NO: 6 encompasses a variant (more specifically *ZRT219X322E325T* variant) encoding *ZRT2* protein in which the amino acid the amino acid at position position19 is different from aspartic acid, the amino acid at position 322 is glutamic acid and the amino acid at position 325 is threonine; a variant (more specifically *ZRT219X322X325T* variant) encoding *ZRT2* protein in which the amino acid at position position19 is different from aspartic acid, the amino acid at position 322 is different from glutamic acid and the amino acid at position 325 is threonine; a variant (more specifically *ZRT219D322X325T* variant) encoding *ZRT2* protein in which the amino acid at position 19 is aspartic acid, the amino acid at position 322 is different from glutamic acid and the amino acid at position 325 is threonine; a variant (more specifically *ZRT219D322X325X* variant) encoding *ZRT2* protein in which the amino acid at position position19 is aspartic acid, the amino acid at position 322 is different from glutamic acid and the amino acid at position 325 is different from threonine; a variant (more specifically *ZRT219D322E325X* variant) encoding *ZRT2* protein in which the amino acid at position position19 is aspartic acid, the amino acid at position 322 is glutamic acid and the amino acid at position 325 is different from threonine; a variant (more specifically *ZRT219D322E325T* variant) encoding *ZRT2* protein in which the amino acid at position position19 is aspartic acid, the amino acid at position 322 is glutamic acid and the amino acid at position 325 is threonine.

In accordance with another alternate embodiment of the present description, the parent strain comprises combinations of variants from the preceding alternate embodiments.

According to an embodiment of the present description, the parent strain comprises at least one of the *YLR125W* gene, hereinafter known as *YLR125X,* encoding an Yrl125w protein in which the amino acid in position 34 is different from lysine; the *SRN2* gene, hereinafter known as *SRN2133X180X,* encoding a *srn2* protein in which the amino acid at position 180 is different from aspartic acid and the amino acid at position 133 is different from glutamic acid; or the *ZRT2* gene, hereinafter known as *ZRT219X322X325X,* encoding a *ZRT2* protein in which the amino acid at position 19 is different from aspartic acid, the amino acid at position 322 is different from glutamic acid and the amino acid at position 325 is different from threonine.

In an embodiment, at least one of the variant of the *YLR125W* polypeptide having the amino acid sequence of SEQ ID NO: 2, the variant of the *SRN2* polypeptide having the amino acid sequence of SEQ ID NO: 4 and/or the variant of the *ZRT2* polypeptide having the amino acid sequence of SEQ ID NO: 6 can be introduced into the parent strain by various methods, well known in themselves to those skilled in the art. In another embodiment, they can be introduced, for example, by crossing the parent strain with a strain which has at least one of the desired *YLR125W34K* variant, *SRN2133E180D* variant and/or *ZRT219D322E325T* variant, and selection from the descendants of this cross, of those to which said allele has been transmitted. Multiples copies of the at least one of the *YLR125W34K* variant, the *SRN2133E180D* variant and/or the *ZRT219D322E325T* variant can be introduced into the parent strain by the same various methods, well known in themselves to those skilled in the art.

In another embodiment, the *YLR125W34K* variant, the *SRN2133E180D* variant and/or the *ZRT219D322E325T* variant can also be introduced by replacement of the initial gene(s) (respectively *YLR125W34X* gene, *SRN2133X180X* gene and/or *ZRT219X322X325X* gene) or in addition to said gene(s), using conventional genetic engineering techniques (cf. for example AMBERG et al., Methods in yeast genetics: a Cold Spring Harbor Laboratory course manual, Cold Spring Harbor Laboratory Press, 2005).

In a further embodiment, the YLR125X gene encodes an Yrl125w protein of SEQ ID NO: 7 in which the amino acid in position 34 is different from lysine; the SRN2133X180X gene encodes a *srn2* protein of SEQ ID NO: 8 in which the amino acid at position 180 is different from aspartic acid and the amino acid at position 133 is different from glutamic acid; and/or the ZRT219X322X325X gene encodes a *ZRT2* protein of SEQ ID NO: 9 in which the amino acid at position 19 is different from aspartic acid, the amino acid at position 322 is different from glutamic acid and the amino acid at position 325 is different from threonine.

Gene inactivation can be used as a preliminary step to allelic replacement. Antibiotic cassette natMX can be used to amplify by high fidelity PCR from appropriate plasmids, with primers containing about 80 bp of upstream and downstream homology arms for the gene to delete, and from 20 to 25 bp targeting the coding sequence of the cassette. The amplified fragment can then be used to transform yeast cells that, replaced the target gene through homologous recombination. Likewise, CRISPR (Clustered Regularly Interspaced Short Palindromic Repeats) technology can be used for targeted genome modifications, namely for removing an antibiotic cassette from one of the parents for the QTL mapping and for all the allelic replacements.

If the method in accordance with the description is carried out using a haploid parent strain carrying the *YLR125W34X* gene, the introduction, into said strain, of a copy of the *YLR125W34K* variant by crossing produces a heterozygous *YLR125W34X*/ *YLR125W34K* strain, producing an amount of hydrogen sulfide which is lower than that produced by the parent strain from which it is derived. It is also possible to obtain haploid descendants of this strain which have the *YLR125W34K* variant and therefore produce low amounts of hydrogen sulfide. By means of the series of backcrosses between descendants having the *YLR125W34K* variant and the parent strain, it is thus possible to obtain a strain with a genome close to that of the parent strain, having acquired the *YLR125W34K* variant and producing low amounts of hydrogen sulfide.

Likewise, if the method in accordance with the description is carried out using a parent strain carrying the *SRN2133X180X* gene, the crossing of said strain with a strain having the *SRN2133E180D* variant produces a heterozygous *SRN2133X180X* / *SRN2133E180D* strain, producing hydrogen sulfide which is lower than that produced by the parent strain from which it is derived. It is also possible, as in the case of *YLR125W34K* variant, to obtain haploid descendants of this strain having the *SRN2133E180D* variant, and by means of backcrosses with the parent strain, to obtain a strain having the *SRN2133E180D* variant on the genetic background of the parent strain.

Furthermore, in accordance with the present description, if the method is carried out using a parent strain carrying the *ZRT219X322X325X* gene, the crossing of said strain with a strain having the *ZRT219D322E325T* variant produces a heterozygous *ZRT219X322X325X* / *ZRT219D322E325T* strain, producing hydrogen sulfide which is lower than that produced by the parent strain from which it is derived. It is also possible, as in the case of *YLR125W* and *SRN2* variants, to obtain haploid descendants of this strain having the *ZRT219D322E325T* variant, and by means of backcrosses with the parent strain, to obtain a strain having the *ZRT219D322E325T* variant on the genetic background of the parent strain.

An embodiment of the present description is also an isolated polynucleotide encoding the variant of the *YLR125W* protein of sequence SEQ ID NO: 2 in which the amino acid in position 34 is lysine and has a C-terminal truncation, which includes the *YLR125W34K* variant. According to one embodiment of the present description, this polynucleotide is defined by the sequence SEQ ID NO: 1. This polynucleotide can be used, in the context of the method in accordance with the description described above, to introduce the *YLR125W34K* variant into a yeast strain.

A further embodiment of the present description is also an isolated polynucleotide encoding the variant of the *SRN2* protein of sequence SEQ ID NO: 4, which includes the *SRN2133E180D* variant. According to one embodiment of the present description, this polynucleotide is defined by the sequence SEQ ID NO: 3. This polynucleotide can be used, in the context of the method in accordance with the description described above, to introduce the *SRN2133E180D* variant into a yeast strain.

In yet a further embodiment of the present description is also an isolated polynucleotide encoding the variant of the *ZRT2* protein of sequence SEQ ID NO: 6, which includes the *ZRT219D322E325T* variant. According to one embodiment of the present description, this polynucleotide is defined by the sequence SEQ ID NO: 5. This polynucleotide can be used, in the context of the method in accordance with the description described above, to introduce the *ZRT219D322E325T* variant into a yeast strain.

### Methods for reducing hydrogen sulfide levels in fermented products

The present description also provides a method for producing fermentation products with reduced hydrogen sulfide wherein the fermentation products have a decreased and/or reduced hydrogen sulfide levels. In an embodiment, the method comprising fermenting a fermentation medium with a host cell comprising at least one of a polynucleotide encoding a variant of the *YLR125W* polypeptide, wherein the variant has the amino acid sequence of SEQ ID NO: 2 and comprises a substitution at position 34 (different from asparagine) and a C-terminal truncation, a polynucleotide encoding a variant of the *SRN2* polypeptide having the amino acid sequence of SEQ ID NO: 4; or a polynucleotide encoding a variant of the *ZRT2* polypeptide having the amino acid sequence of SEQ ID NO: 6 , wherein the fermentation products have a reduced hydrogen sulfide.

The present description further provides a fermentation product comprising reduced hydrogen sulfide levels comprising a host cell comprising at least one of a polynucleotide encoding a variant of the *YLR125W* polypeptide, wherein the variant has the amino acid sequence of SEQ ID NO: 2 and comprises a substitution at position 34 (different from asparagine) and a C-terminal truncation, a polynucleotide encoding a variant of the *SRN2* polypeptide having the amino acid sequence of SEQ ID NO: 4, or a polynucleotide encoding a variant of the *ZRT2* polypeptide having the amino acid sequence of SEQ ID NO: 6, wherein the fermentation product has a reduced hydrogen sulfide.

In an embodiment of the method and the fermentation product, the polynucleotide encodes a variant of the *YLR125W* polypeptide, wherein the variant has the amino acid sequence of SEQ ID NO: 2 and comprises a substitution at position 34 (different from asparagine) and a C-terminal truncation. In another embodiment, the polynucleotide encodes a variant of the *YLR125W* polypeptide having the amino acid sequence of SEQ ID NO: 2 producing reduced levels of hydrogen sulfide, and comprises a substitution at position 34 (different from asparagine) and a C-terminal truncation. In a further embodiment, the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 (different from asparagine) and a C-terminal truncation. In yet a further embodiment, the polynucleotide encodes a variant of the *YLR125W* polypeptide having the amino acid sequence of SEQ ID NO: 2 producing reduced levels of hydrogen sulfide, and comprises a substitution at position 34 that is lysine and a C-terminal truncation. In yet another embodiment, the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 that is lysine and a C-terminal truncation. In yet another embodiment, the polynucleotide encoding the variant of the *YLR125W* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO:1.

In an embodiment of the method and the fermentation product, the polynucleotide encodes a variant of the *SRN2* polypeptide and the variant has the amino acid sequence of SEQ ID NO: 4 and comprises substitutions at position 180 (different from asparagine) and at position 133 (different from glycine). In another embodiment, the polynucleotide encodes the variant of the *SRN2* polypeptide having the amino acid sequence of SEQ ID NO: 4 producing reduced levels of hydrogen sulfide, and comprises substitutions at position 180 (different from asparagine) and at position 133 (different from glycine). In a further embodiment, the variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 180 (different from asparagine) and at position 133 (different from glycine). In yet a further embodiment, the polynucleotide encodes the variant of the *SRN2* polypeptide having the amino acid sequence of SEQ ID NO: 4 producing reduced levels of hydrogen sulfide, and comprises substitutions at position 133 and 180 that are respectively glutamic acid and aspartic acid. In yet another embodiment, the variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 and 180 that are respectively glutamic acid and aspartic acid. In an embodiment, the variant of the SRN2 polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.

In an embodiment, the polynucleotide encodes a variant of the *ZRT2* polypeptide and the variant has the amino acid sequence of SEQ ID NO: 6 and comprises substitutions at position 19 (different from glycine), at position 322 (different from lysine), and at position 325 (different from methionine). In another embodiment, the polynucleotide encodes the variant of the *ZRT2* polypeptide having the amino acid sequence of SEQ ID NO: 6 producing reduced levels of hydrogen sulfide, and comprises substitutions at position 19 (different from glycine), at position 322 (different from lysine), and at position 325 (different from methionine). In a further embodiment, the variant of the *Saccharomyces ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position 19 (different from glycine), at position 322 (different from lysine), and at position 325 (different from methionine). In yet a further embodiment, the polynucleotide encodes the variant of the *ZRT2* polypeptide having the amino acid sequence of SEQ ID NO: 6 producing reduced levels of hydrogen sulfide, and comprising substitution at position19, 322 and 325 that are respectively aspartic acid, glutamic acid and threonine. In yet another embodiment, the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position19, 322 and 325 that are respectively aspartic acid, glutamic acid and threonine. In an embodiment the polynucleotide encoding a variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.

In an embodiment, the fermentation product having decreased and/or reduced levels of hydrogen sulfide is produced, and the fermentation product is a beverage. In another embodiment, the fermentation medium is a juice, a must or a wort. In a further embodiment, the fermentation product is a grape juice must. In yet a further embodiment, the fermentation products are wine, beer, cider or champagne.

In any of the preceding embodiments, the host cell is a yeast cell, for example, a *Saccharomyces cerevisiae, Kluyveromyces lactis, Yarowia lipolytica,* or *Schizosaccharomyces pombe* yeast cell. Yeast cells used in the production of fermented beverages, e.g., wine, port, Madeira, beer, champagne, etc. (e.g., "wine yeast," "beer yeast," "champagne yeast," etc.) find use for the introduction of a at least one of a nucleic acid encoding an exogenous hydrogen sulfide reducing polypeptide of the present description. In some embodiments, the yeast cell strain is a *Saccharomyces cerevisiae* strain. In some embodiments, the *Saccharomyces cerevisiae* yeast cell strain is a wine yeast.

In any of the preceding embodiments, the polynucleotides are from a *Saccharomyces cerevisiae, Kluyveromyces lactis, Yarowia lipolytica,* or *Schizosaccharomyces pombe* yeast cell. Yeast cells used in the production of fermented beverages. In some embodiments, the polynucleotides are from a *Saccharomyces cerevisiae* strain.

### EXAMPLES

The following examples are offered to illustrate, but not to limit the claimed invention.

### EXAMPLE 1

### Effect of the variants of the YLR125W Gene in the Production of Hydrogen Sulfide

The *YLR125W* gene variant present in the *Saccharomyces cerevisiae* JN20 strain (*YLR125W* JN20) encodes an *YLR125W* protein in which the amino acid in position 34 is an asparagine, whereas the variant present in the JN27 strain (*YLR125W* JN27) encodes an *Ylr125w* protein in which the amino acid at position 34 is lysine and which has a C-terminal truncation.

Firstly, the *YLR125W* gene was inactivated in each of the JN20 and JN27 haploid parental strains, by insertion of the antibiotic cassette natMX, so as to obtain respectively the JN20*ylr125w*Δ::natMX strains and the JN27*ylr125w*Δ::natMX strain.

The *ylr125w*Δ::natMX strains of the JN20 and JN27 parental strains, were then transformed with a combination of a plasmid with a RNA guide targeting the natMX, and a repair fragment corresponding to the variant of the other parental strain so as to obtain respectively the haploid strains JN27 - *YLR125W* ^{JN20} and JN20 - *YLR125W* ^{JN27}. The production of hydrogen sulfide by these strains which contain the variant of *YLR125W* of the other parental strain were evaluated under enological alcoholic fermentation conditions.

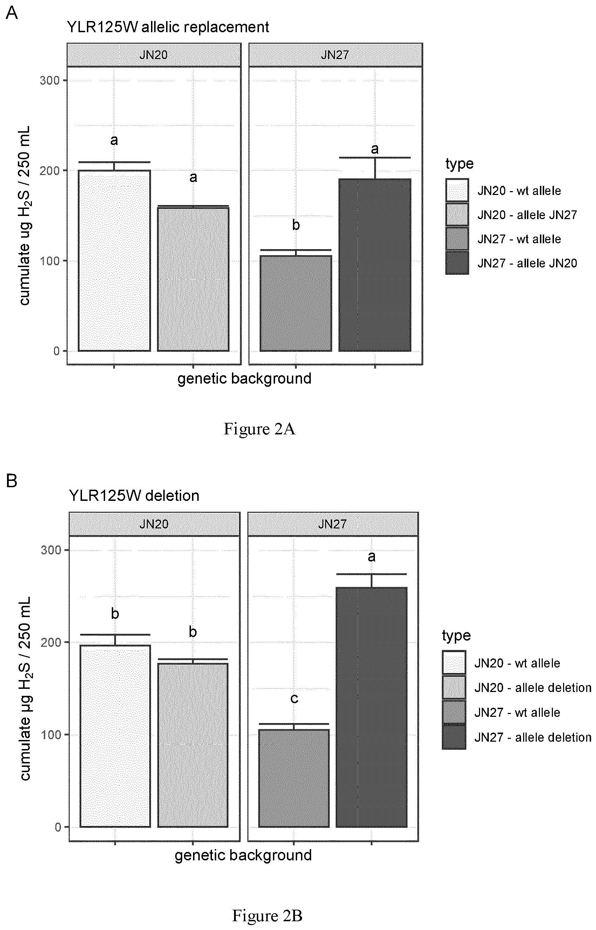

*YLR125W,* whose function is unknown, was present in a truncated form in the genome of JN27. The replacement of the JN20 variant of *YLR125W* with the truncated form of JN27 led to a 21% decrease in hydrogen sulfide production, while the replacement of the JN27 variant by the full-length version of JN20 led to an increase of 81% (Figure 2A). As this phenotype could result from the inactivation of the short variant, we compared the result of the variant exchange with that of strains deleted for *YLR125W.* Surprisingly, the effect of the deletion of *YLR125W* gene was only visible in the JN27 genetic background, with an almost 2.5 times higher production of hydrogen sulfide for the JN27*ylr125w*Δ::natMX strain while the production of hydrogen sulfide of JN27 strain carrying JN20 variant produced two time more hydrogen sulfide (Fig. 2B).

It is noted that the production of the hydrogen sulfide content is lower when the *YLR125W*JN27 variant is active than when the variant is the one derived from the JN20 strain. The *YLR125W*JN27 variant therefore results in a reduction in hydrogen sulfide.

### EXAMPLE 2

### Effect of the variant of the SRN2 Gene in the Production of Hydrogen Sulfide

The *SRN2* gene variant present in the *Saccharomyces cerevisiae* JN20 strain) (*SRN*2JN20) encodes a srn2 protein in which the amino acid at position 180 is asparagine and the amino acid at position 133 is glycine, whereas the variant present in the JN27 strain (*SRN2*JN27) encodes a srn2 protein in which the amino acid at position 180 is aspartic acid and the amino acid at position 133 is glutamic acid.

Firstly, the *SRN2* gene was inactivated in each of the JN20 and JN27 parental strains, by insertion of the antibiotic cassette natMX, so as to obtain respectively the JN20*srn2*Δ::natMX strains and the JN27*srn2*Δ::natMX strain.

The *srn2*Δ::natMX strains of the JN20 and JN27 parental strains, were then transformed with the combination of a plasmid and the RNA guide targeting the natMX, and a repair fragment corresponding to the variant of the other parental strain so as to obtain respectively the haploid strains JN27 - *SRN2* ^{JN20} and JN20 - *SRN2* ^{JN27}. The production of hydrogen sulfide by these strains which contain the variant of *SRN2* of the other parental strain were evaluated under enological alcoholic fermentation conditions.

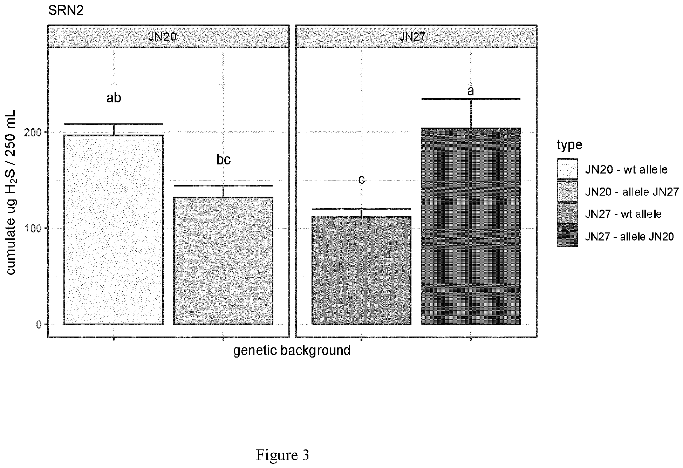

The production of hydrogen sulfide by these strains is shown in FIG. 3: production of hydrogen sulfide. The replacement of JN20 variant by JN27 variant decreased hydrogen sulfide production by 33%, while the replacement of the JN27 variant by that of JN20 increased this production by 83%.

It is noted that the production of the hydrogen sulfide content is lower when the *SRN2*JN27 variant is active than when the variant is the one derived from the JN20 strain. The *SRN2*JN27 variant therefore results in a reduction in hydrogen sulfide.

### EXAMPLE 3

### Effect of the variant of the ZRT2 Gene in the Production of Hydrogen Sulfide

The *ZRT2* gene variant present in the *Saccharomyces cerevisiae* JN20 strain (ZRT2JN20) encodes a *Zrt2* protein in which the amino acid at position 19 is glycine, the amino acid at position 322 is lysine and the amino acid at position 325 is methionine, whereas the variant present in the JN27 strain (*ZRT2*JN27) encodes a *Zrt2* protein in which the amino acid at position 19 is aspartic acid, the amino acid at position 322 is glutamic acid and the amino acid at position 325 is threonine.

Firstly, the *ZRT2* gene was inactivated in each of the JN20 and JN27 haploid parental strains, by insertion of the antibiotic cassette natMX, so as to obtain respectively the JN20*zrt2*Δ::natMX strains and the JN27*zrt2*Δ::natMX strain.

The *zrt2*Δ::natMX strains of the JN20 and JN27 parental strains, were then transformed with a combination of a plasmid with a RNA guide targeting the natMX, and a repair fragment corresponding to the variant of the other parental strain so as to obtain the haploid strains JN20 - *ZRT2* ^{JN27} and JN27 - *ZRT2* ^{JN20} respectively. The production of hydrogen sulfide by these strains which contain the variant of *ZRT2* of the other parental strain were evaluated under enological alcoholic fermentation conditions.

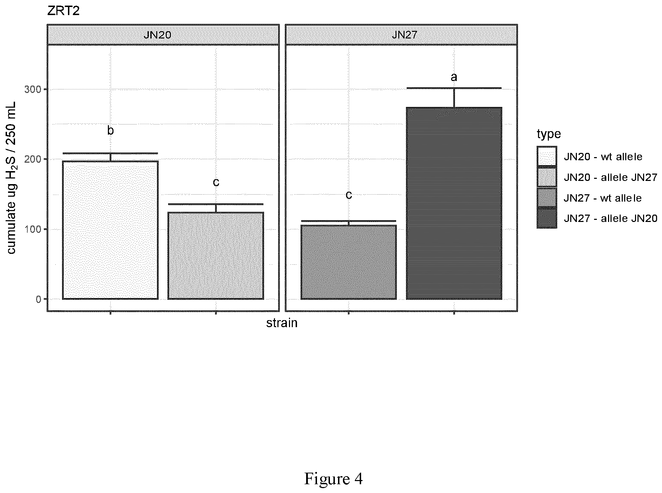

The results are shown in Figure 4: production of hydrogen sulfide. The JN27 variant in JN20 decreased the hydrogen sulfide production by 37%, whereas JN20 variant increased hydrogen sulfide production by 160% in JN27 (Figure 4).

It is noted that the production of the hydrogen sulfide content is lower when the *ZRT2*JN27 variant is active than when the variant is the one derived from the JN20 strain. The *ZRT2*JN27 variant therefore results in a reduction in hydrogen sulfide.

### EXAMPLE 4

### Combined Effect of the variant of the YLR125W, SRN2 and/or ZRT2 Gene on the Production of Hydrogen Sulfide

The impact of a combination of the three variant forms of *YLR125W*^{JN27}*, SRN2*^{JN27} and *ZRT2*^{JN27} was evaluated in strain JN20 and the combination of the three variant forms of *YLR125W*^{JN20}, *SRN2*^{JN20} and *ZRT2*^{JN20} in strain JN27.

Firstly, the region encompassing *SRN2* and *YLR125W* was inactivated in each of the JN20 - *ZRT2* ^{JN27} and JN27 *- ZRT2* ^{JN20} haploid strains, by insertion of the antibiotic cassette natMX, so as to obtain respectively the JN20 *ZRT2*^{JN27}-*ylr125w*Δ::natMX strains and the JN27 *ZRT2*^{JN20}*srn2-ylr125w*Δ::natMX strain.

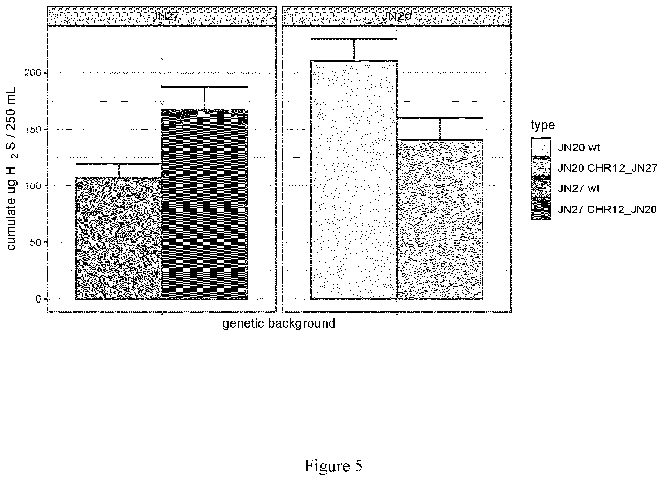

Each of the deletion strains were then transformed with a combination of a plasmid with a RNA guide targeting the natMX, and a repair fragment corresponding to the variants of the other parental strain so as to obtain the haploid strains JN27 - *ZRT2* ^{JN20} *SRN2-YLR125W* ^{JN20} and JN20 - *ZRT2* ^{JN27} *SRN2-YLR125W* ^{JN27} respectively. The production of hydrogen sulfide by these strains which contain the variants of *ZRT2, SRN2* and *YLR125W* of the other parental strain were evaluated under enological alcoholic fermentation conditions. The results are shown in Figure. 5: production of hydrogen sulfide. The JN27 variants in JN20 decreased the hydrogen sulfide production by 33%, whereas JN20 variants increased hydrogen sulfide production by 157% in JN27 (Figure 5).

While the invention has been described in connection with specific embodiments thereof, it will be understood that the scope of the claims should not be limited by the preferred embodiments set forth in the examples, but should be given the broadest interpretation consistent with the description as a whole.

SEQUENCE LISTING

SEQ ID NO: 1 (YLR125W_JN27):

ATGGGAGAAATTTTAGAACTAACTAATAAGAATTTCATGAGCCACTTGAAAAAGGACATAA
CTTCGCAAGAATCCTTGAAAAGTAGAATTGAGGATAAAAAATGGTGA

SEQ ID NO: 2 (YLR125W_JN27):

MGEILELTNKNFMSHLKKDITSQESLKSRIEDKKW

SEQ ID NO: 3 (SRN2_JN27):

ATGGATGTGGTACCATCTCCGGGTTTACCAGAAAAAGTAAACGAAAAAAGCAAAAATATTC
CCTTACCTGAAGGAATTAACCTTTTATCATCAAAGGAGATAATCGATCTAATTCAAACCCA
CAGACACCAATTAGAGTTATACGTCACAAAATTCAATCCCTTAACGGACTTTGCTGGAAAG
ATTCATGCATTCAGAGATCAATTTAAACAATTGGAAGAAAATTTTGAAGATTTACATGAGC
AAAAAGATAAGGTCCAAGCCTTATTAGAAAACTGTAGGATTTTAGAATCCAAATATGTAGC
TAGCTGGCAAGATTACCATTCCGAATTTTCAAAAAAATATGGTGACATAGCATTAAAAAAG
AAATTAGAACAAAACACAAAGAAACTTGATGAGGAGAGTTCACAGCTAGAAACTACCACAA
GGTCCATTGACTCAGCCGATGACCTGGATCAGTTCATCAAAAATTATCTGGACATACGAAC
ACAATATCACTTGAGGAGAGAGAAACTGGCGACCTGGGATAAACAAGGTGATTTGAAATAC
TAG

SEQ ID NO: 4 (SRN2_JN27):

MDVVPSPGLPEKVNEKSKNIPLPEGINLLSSKEIIDLIQTHRHQLELYVTKFNPLTDFAGK
IHAFRDQFKQLEENFEDLHEQKDKVQALLENCRILESKYVASWQDYHSEFSKKYGDIALKK
KLEQNTKKLDEESSQLETTTRSIDSADDLDQFIKNYLDIRTQYHLRREKLATWDKQGDLKY
-

SEQ ID NO: 5 (ZRT2_JN27):

ATGGTTGATCTTATAGCGAGGGATGACTCCGTAGATACTTGCCAAGCTTCTAACGACTACA
ATGGGCACGCAGGTCTTAGAATTCTGGCAGTATTCATTATACTGATATCGTCAGGATTGGG
AGTTTATTTCCCAATTTTGTCATCACGGTATTCGTTTATAAGGCTACCAAATTGGTGCTTT
TTCATAGCGAAGTTCTTCGGTTCTGGTGTCATTGTTGCCACAGCGTTCGTTCATCTTCTAC
AGCCCGCAGCCGAAGCTCTGGGAGATGAATGTCTTGGTGGCACATTTGCCGAATATCCATG
GGCTTTTGGGATCTGTTTAATGTCGCTTTTCTTACTTTTCTTCACTGAAATCATCACGCAT
TATTTTGTAGCGAAAACGCTGGGACACGATCATGGGGACCATGGGGAAGTTACCAGTATTG
ATGTTGATGCTCCCAGTTCGGGATTTGTCATCAGAAATATGGACTCGGATCCTGTATCTTT
CAATAACGAAGCTGCCTACTCCATCCATAATGACAAAACTCCGTACACTACTAGAAATGAA
GAGATTGTCGCTACTCCTATAAAGGAAAAAGAACCCGGCTCAAATGTTACTAATTATGATC
TGGAACCGGGAAAAACAGAGTCACTAGCTAATGAACTAGTTCCAACCAGTTCCCATGCGAC
AAATCTCGCTTCTGTACCTGGAAAAGATCATTATTCTCACGAAAATGACCATCAAGATGTC
TCCCAGTTGGCTACACGTATCGAGGAGGAAGATAAAGAGCAGTATCTCAATCAGATACTAG

CTGTTTTTATTCTAGAATTTGGCATCATCTTTCACTCTGTATTTGTGGGTCTTTCGCTATC
TGTCGCGGGTGAAGAATTCGAAACCTTATTTATCGTTTTAACTTTCCACCAAATGTTCGAA
GGTTTGGGTCTAGGCACAAGAGTTGCCGAAACGAATTGGCCAGAAAGTGAGAAGTACACGC
CTTGGTTAATGGGATTAGCCTTCACTTTAACGTCACCCATAGCAGTCGCGGTAGGTATTGG
TGTCAGACACTCTTGGATACCTGGCTCTAGAAGAGCATTAATTGCTAATGGTGTTTTTGAC
TCGATATCATCAGGAATTCTTATTTATACTGGACTAGTCGAATTAATGGCTCATGAATTCT
TATACTCTAATCAATTCAAAGGACCTGATGGCCTCAAAAAAATGCTTAGTGCATATCTCAT
CATGTGTTGTGGAGCTGCTTTAATGGCTCTTCTAGGGAAATGGGCATAG

SEQ ID NO: 6 (ZRT2_JN27):

MVDLIARDDSVDTCQASNDYNGHAGLRILAVFIILISSGLGVYFPILSSRYSFIRLPNWCF
FIAKFFGSGVIVATAFVHLLQPAAEALGDECLGGTFAEYPWAFGICLMSLFLLFFTEIITH
YFVAKTLGHDHGDHGEVTSIDVDAPSSGFVIRNMDSDPVSFNNEAAYSIHNDKTPYTTRNE
EIVATPIKEKEPGSNVTNYDLEPGKTESLANELVPTSSHATNLASVPGKDHYSHENDHQDV
SQLATRIEEEDKEQYLNQILAVFILEFGIIFHSVFVGLSLSVAGEEFETLFIVLTFHQMFE
GLGLGTRVAETNWPESEKYTPWLMGLAFTLTSPIAVAVGIGVRHSWIPGSRRALIANGVFD
SISSGILIYTGLVELMAHEFLYSNQFKGPDGLKKMLSAYLIMCCGAALMALLGKWA

SEQ ID NO: 7 (YLR125W_JN20):

MGEILELTNKNFMSHLKKDITSQESLKSRIEDKNGDVASPKEDNYPLLNETAAWPDGVITS
EEGCSSSGEKENSGLCSEESSEEDPEEAEEESARAFGELVAVLRDKDIPLNVLDEPQMKDW
LEKYTGVYRSSWHG

SEQ ID NO: 8 (SRN2_JN20):

MDVVPSPGLPEKVNEKSKNIPLPEGINLLSSKEIIDLIQTHRHQLELYVTKFNPLTDFAGK
IHAFRDQFKQLEENFEDLHEQKDKVQALLENCRILESKYVASWQDYHSEFSKKYGDIALKK
KLEQNTKKLDGESSQLETTTRSIDSADDLDQFIKNYLDIRTQYHLRREKLATWDKQGNLKY

SEQ ID NO: 9 (ZRT2 JN20):

MVDLIARDDSVDTCQASNGYNGHAGLRILAVFIILISSGLGVYFPILSSRYSFIRLPNWCF
FIAKFFGSGVIVATAFVHLLQPAAEALGDECLGGTFAEYPWAFGICLMSLFLLFFTEIITH
YFVAKTLGHDHGDHGEVTSIDVDAPSSGFVIRNMDSDPVSFNNEAAYSIHNDKTPYTTRNE
EIVATPIKEKEPGSNVTNYDLEPGKTESLANELVPTSSHATNLASVPGKDHYSHENDHQDV
SQLATRIEEEDKEQYLNQILAVFILEFGIIFHSVFVGLSLSVAGEEFETLFIVLTFHQMFE
GLGLGTRVAETNWPESKKYMPWLMGLAFTLTSPIAVAVGIGVRHSWIPGSRRALIANGVFD
SISSGILIYTGLVELMAHEFLYSNQFKGPDGLKKMLSAYLIMCCGAALMALLGKWA

### FURTHER ASPECTS OF THE INVENTION:

1. A method for producing fermentation products with reduced hydrogen sulfide, the method comprising fermenting a fermentation medium with a host cell comprising at least one of
   a polynucleotide encoding a variant of the *YLR125W* polypeptide, wherein the variant has the amino acid sequence of SEQ ID NO: 2 and comprises a substitution at position 34 (different from asparagine) and a C-terminal truncation,
   a polynucleotide encoding a variant of the *SRN2* polypeptide, wherein the variant has the amino acid sequence of SEQ ID NO: 4 and comprises substitutions at position 133 (different from glycine) and at position 180 (different from asparagine), or
   a polynucleotide encoding a variant of *ZRT2* polypeptide, wherein the variant has the amino acid sequence of SEQ ID NO: 6 and comprises substitutions at position 19 (different from glycine), at position 322 (different from lysine), and at position 325 (different from methionine),
   wherein the fermentation products have a reduced hydrogen sulfide.
2. The method of aspect 1, wherein the polynucleotide encoding the variant of the *YLR125W* polypeptide having the amino acid sequence of SEQ ID NO: 2 produces reduced levels of hydrogen sulfide, and comprises a substitution the amino acid at position 34 of the *YLR125W* polypeptide (different from asparagine) and a C-terminal truncation.
3. The method of aspect 2, wherein the polynucleotide encodes the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 (different from asparagine) and a C-terminal truncation.
4. The method of aspect 1, wherein the polynucleotide encodes a variant of the *Saccharomyces YLR125W* polypeptide having the amino acid sequence of SEQ ID NO: 2 producing reduced levels of hydrogen sulfide, and comprises a substitution the amino acid at position 34 of the *YLR125W*polypeptide (lysine) and a C-terminal truncation.
5. The method of aspect 4, wherein the polynucleotide encodes the variant of the *YLR125W* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution the amino acid at position 34 that is lysine and a C-terminal truncation.
6. The method of any one of aspect 1 to 5, wherein the polynucleotide encoding the variant of the *YLR125W* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 1.
7. The method of aspect 1, wherein the polynucleotide encodes a variant of the *SRN2* polypeptide, wherein the variant has the amino acid sequence of SEQ ID NO: 4 and comprises substitutions at position 133 (different from glycine) and at position 180 (different from asparagine).
8. The method of aspect 7, wherein the polynucleotide encodes the variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 (different from glycine) and at position 180 (different from asparagine).
9. The method of aspect 1, wherein the polynucleotide encodes the variant of the *SRN2* polypeptide having the amino acid sequence of SEQ ID NO: 4 producing reduced levels of hydrogen sulfide, and comprises substitutions at position 133 and 180 that are respectively glutamic acid and aspartic acid.
10. The method of aspect 9, wherein the polynucleotide encodes a variant of the *SRN2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 and 180 that are respectively glutamic acid and aspartic acid.
11. The method of any one of aspects 1 and 7 to 10, wherein the polynucleotide encoding the variant of the *SRN2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.
12. The method of aspect 1, wherein the polynucleotide encodes the variant of the *ZRT2* polypeptide having the amino acid sequence of SEQ ID NO: 6 producing reduced levels of hydrogen sulfide, and comprising substitutions position 19 (different from glycine), at position 322 (different from lysine) and at position 325 (different from methionine).
13. The method of aspect 12, wherein the polynucleotide encodes the variant of the *ZRT2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprising substitutions at position 19 (different from glycine), at position 322 (different from is not lysine) and at position 325 (different from methionine).
14. The method of aspect 1, wherein the polynucleotide encodes the variant of the *ZRT2* polypeptide having the amino acid sequence of SEQ ID NO: 6 producing reduced levels of hydrogen sulfide, and comprising substitutions at position19 that is aspartic acid, at position 322 that is glutamic acid and at position 325 that is threonine.
15. The method of aspect 14, wherein the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprising substitutions at position 19 that is aspartic acid, at position 322 that is glutamic acid and at position 325 that is threonine.
16. The method of any one of aspects 1 and 12 to 15, wherein the polynucleotide encoding a variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.
17. The method of any one of aspects 1 to 16, wherein a fermentation product having reduced levels of hydrogen sulfide is produced, wherein the fermentation product is a beverage.
18. The method of any one of aspects 1 to 16, wherein the fermentation medium is a juice, a must or a wort.
19. The method of aspect 18, wherein the fermentation product is a grape juice must.
20. The method of any one of aspects 1 to 17, wherein the fermentation products are wine, beer, cider or champagne.
21. An improved host cell culture that produces reduced levels of hydrogen sulfide comprising a population of yeast cells, the yeast cells comprising at least one of
   an exogenous polynucleotide encoding a variant of the *YLR125W* polypeptide, wherein the variant has the amino acid sequence of SEQ ID NO: 2 and comprises a substitution at position 34 (different from asparagine) and a C-terminal truncation,
   an exogenous polynucleotide encoding a variant of the *SRN2* polypeptide, wherein the variant has the amino acid sequence of SEQ ID NO: 4 and comprises substitutions at position 133 (different from glycine) and at position 180 (different from asparagine), or
   an exogenous polynucleotide encoding a variant of the *ZRT2* polypeptide, wherein the variant has the amino acid sequence of SEQ ID NO: 6 and comprises substitutions at position 19 (different from glycine), at position 322 (different from lysine), and at position 325 (different from methionine),
   wherein the improved host cell culture produces reduced hydrogen sulfide in comparison to a culture of parent cells.
22. The improved host cell culture of aspect 21, wherein the exogenous polynucleotide encodes a variant *YLR125W* polypeptide having the amino acid sequence of SEQ ID NO: 2 producing reduced levels of hydrogen sulfide, and comprises a substitution at position 34 (different from asparagine) and a C-terminal truncation.
23. The improved host cell culture of aspect 22, wherein the variant of the *YLR125W* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 (different from asparagine) and a C-terminal truncation.
24. The improved host cell culture of aspect 21, wherein the polynucleotide encodes a variant of the *YLR125W* polypeptide having the amino acid sequence of SEQ ID NO: 2 producing reduced levels of hydrogen sulfide, and comprises a substitution at position 34 (lysine) and a C-terminal truncation.
25. The improved host cell culture of aspect 24, wherein the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 (lysine) and a C-terminal truncation.
26. The improved host cell culture of any one of aspect 21 to 25, wherein the polynucleotide encoding the variant of the *YLR125W* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO:1.
27. The improved host cell culture of aspect 21, wherein the polynucleotide encodes the variant of the *SRN2* polypeptide having the amino acid sequence of SEQ ID NO: 4 producing reduced levels of hydrogen sulfide, and comprises substitutions at position 180 (different from asparagine) and at position 133 (different from glycine).
28. The improved host cell culture of aspect 27, wherein variant of the *SRNSRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 180 (different from asparagine) and at position 133 (different from glycine).
29. The improved host cell culture of aspect 21, wherein the polynucleotide encodes the variant of the *SRN2* polypeptide having the amino acid sequence of SEQ ID NO: 4 producing reduced levels of hydrogen sulfide, and comprising substitutions at position 133 and 180 that are respectively glutamic acid and aspartic acid.
30. The improved host cell culture of aspect 29, wherein the variant of the *SRNSRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprising substitution at position 133 and 180 that are respectively glutamic acid and aspartic acid.
31. The improved host cell culture of any one of aspects 21 and 27 to 30, wherein the polynucleotide encoding the variant of the *SRN2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.
32. The improved host cell culture of aspect 21, wherein the polynucleotide encodes the variant of the *ZRT2* polypeptide having the amino acid sequence of SEQ ID NO: 6 producing reduced levels of hydrogen sulfide, and comprises substitutions at position 19 (different from glycine), at position 322 (different from lysine), and at position 325 (different from methionine).
33. The improved host cell culture of aspect 32, wherein the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position 19 (different from glycine), at position 322 (different from lysine), and at position 325 (different from methionine).
34. The improved host cell culture of aspect 21, wherein the polynucleotide encodes the variant of the *ZRT2* polypeptide having the amino acid sequence of SEQ ID NO: 6 producing reduced levels of hydrogen sulfide, and comprising substitutions at positions 19, 322 and 325 that are respectively aspartic acid, glutamic acid and threonine.
35. The improved host cell culture of aspect 34, wherein the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprising substitutions at positions 19, 322 and 325 that are respectively aspartic acid, glutamic acid and threonine.
36. The improved host cell culture of any one of aspects 21 and 32 to 35, wherein the polynucleotide encoding a variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.
37. A method of producing an improved host cell that produces reduced levels of hydrogen sulfide, the method comprising at least one step of
   replacing an endogenous polynucleotide encoding a hydrogen sulfide producing *YLR125W* polypeptide with a polynucleotide encoding a hydrogen sulfide reducing variant of the *YLR125W* polypeptide by introducing into a parent of the host cell the polynucleotide encoding the hydrogen sulfide reducing variant of the *YLR125W* polypeptide, wherein the variant has the amino acid sequence of SEQ ID NO: 2 and comprises a substitution at position 34 (different from asparagine) and a C-terminal truncation,
   replacing an endogenous polynucleotide encoding a hydrogen sulfide producing *SRN2* polypeptide with a polynucleotide encoding a hydrogen sulfide reducing variant of the *SRN2* polypeptide by introducing into a parent of the host cell the polynucleotide encoding the hydrogen sulfide reducing variant of the *SRN2* polypeptide, wherein the variant has the amino acid sequence of SEQ ID NO: 4 and comprises substitutions at position 133 (different from glycine) and at position 180 (different from asparagine), or
   replacing an endogenous polynucleotide encoding a hydrogen sulfide producing *ZRT2* polypeptide with a polynucleotide encoding a hydrogen sulfide reducing variant of the *ZRT2* polypeptide by introducing into a parent of the host cell the hydrogen sulfide reducing polynucleotide encoding the variant of the *ZRT2* polypeptide, wherein the variant has the amino acid sequence of SEQ ID NO: 6 and comprises substitutions at position 19 (different from glycine), at position 322 (different from lysine), and at position 325 (different from methionine),
   wherein the parent of the improved host cell produces hydrogen sulfide.
38. The method of aspect 37, wherein the polynucleotide encodes the hydrogen sulfide reducing variant of the *YLR125W* polypeptide having the amino acid sequence of SEQ ID NO: 2 producing reduced levels of hydrogen sulfide, and comprises a substitution at position 34 (different from asparagine) and a C-terminal truncation.
39. The method of aspect 38, wherein the hydrogen sulfide reducing variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid of SEQ ID NO: 2, and comprises a substitution at position 34 (different from asparagine) and a C-terminal truncation.
40. The method of aspect 37, wherein the polynucleotide encodes the hydrogen sulfide reducing variant of the *YLR125W* polypeptide having the amino acid sequence of SEQ ID NO: 2 producing reduced levels of hydrogen sulfide, and comprises a substitution at position 34 that is lysine and a C-terminal truncation.
41. The method of aspect 40, wherein the hydrogen sulfide reducing variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid of SEQ ID NO: 2, and comprises a substitution at position 34 that is lysine and a C-terminal truncation.
42. The method of aspect 37 to 41, wherein the polynucleotide encoding the hydrogen sulfide reducing variant of the *YLR125W polypeptide* shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 1.
43. The method of aspect 37, wherein the polynucleotide encodes the hydrogen sulfide reducing variant of the *SRN2* polypeptide having the amino acid sequence of SEQ ID NO: 4 producing reduced levels of hydrogen sulfide, and comprises substitutions at position 133 (different from glycine) and at position 180 (different from asparagine).
44. The method of aspect 43, wherein the hydrogen sulfide reducing variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a SRN2 of SEQ ID NO: 4, and comprises substitutions at position 133 (different from glycine) and at position 180 (different from asparagine).
45. The method of aspect 37, wherein the polynucleotide encodes the hydrogen sulfide reducing variant of the *SRN2* polypeptide having the amino acid sequence of SEQ ID NO: 4 producing reduced levels of hydrogen sulfide, and comprises substitutions at position 133 and 180 that are respectively glutamic acid and aspartic acid.
46. The method of aspect 45, wherein the hydrogen sulfide reducing variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a SRN2 of SEQ ID NO: 4, and comprises substitutions at position 133 and 180 that are respectively glutamic acid and aspartic acid.
47. The method of any one of aspects 37 and 43 to 46, wherein the polynucleotide encoding the hydrogen sulfide reducing variant of the *SRN2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.
48. The method of aspect 37, wherein the polynucleotide encodes the hydrogen sulfide reducing variant of the *ZRT2* polypeptide having the amino acid sequence of SEQ ID NO: 6 producing reduced levels of hydrogen sulfide, and comprises substitutions at position 19 (different from glycine), at position 322 (different from lysine), and at position 325 (different from methionine).
49. The method of aspect 48, wherein the hydrogen sulfide reducing variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a *ZRT2* of SEQ ID NO: 4, and comprises substitutions at position 19 (different from glycine), at position 322 (different from lysine), and at position 325 (different from methionine).
50. The method of aspect 37, wherein the polynucleotide encodes the hydrogen sulfide reducing variant of the *ZRT2* polypeptide having the amino acid sequence of SEQ ID NO: 6 producing reduced levels of hydrogen sulfide, and comprises substitutions at position 19, 322 and 325 that are respectively aspartic acid, glutamic acid and threonine.
51. The method of aspect 50, wherein the hydrogen sulfide reducing variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with a *ZRT2* of SEQ ID NO: 4, and comprises substitutions at position 19, 322 and 325 that are respectively aspartic acid, glutamic acid and threonine.
52. The method of any one of aspects 37 and 48 to 51, the polynucleotide encoding a hydrogen sulfide reducing variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.
53. A fermentation product comprising reduced hydrogen sulfide levels comprising a host cell comprising at least one of
   a polynucleotide encoding a variant of the *YLR125W* polypeptide, wherein the variant has the amino acid sequence of SEQ ID NO: 2 and comprises a substitution at position 34 (different from asparagine) and a C-terminal truncation,
   a polynucleotide encoding a variant of the *SRN2* polypeptide, wherein the variant has the amino acid sequence of SEQ ID NO: 4 and comprises substitutions at position 133 (different from glycine) and at position 180 (different from asparagine), or
   a polynucleotide encoding a variant of the *ZRT2* polypeptide, wherein the variant has the amino acid sequence of SEQ ID NO: 6 and comprises substitutions at position 19 (different from glycine), at position 322 (different from lysine), and at position 325 (different from methionine),
   wherein the fermentation product has a reduced hydrogen sulfide.
54. The fermentation product of aspect 53, wherein the polynucleotide encodes a variant of the *YLR125W* polypeptide having the amino acid sequence of SEQ ID NO: 2 producing reduced levels of hydrogen sulfide, and comprises a substitution at position 34 (different from asparagine) and a C-terminal truncation.
55. The fermentation product of aspect 54, wherein the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 (different from asparagine) and a C-terminal truncation.
56. The fermentation product of aspect 53, wherein the polynucleotide encodes a variant of the *YLR125W* polypeptide having the amino acid sequence of SEQ ID NO: 2 producing reduced levels of hydrogen sulfide, and comprises a substitution at position 34 that is lysine and a C-terminal truncation
57. The fermentation product of aspect 56, wherein the variant of the *YLR125W* polypeptide having the amino acid sequence of SEQ ID NO: 2, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a substitution at position 34 that is lysine and a C-terminal truncation.
58. The fermentation product of any one of aspect 53 to 57, wherein the polynucleotide encoding the variant of the *YLR125W* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 1.
59. The fermentation product of aspect 53, wherein the polynucleotide encodes the variant of the *SRN2* polypeptide having the amino acid sequence of SEQ ID NO: 4 producing reduced levels of hydrogen sulfide, and comprises substitutions at position 180 (different from asparagine) and at position 133 (different from glycine).
60. The fermentation product of aspect 59, wherein the variant of the *SRNSRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 (different from glycine) and at position 180 (different from asparagine).
61. The fermentation product of aspect 53, wherein the polynucleotide encodes the variant of the *SRN2* polypeptide having the amino acid sequence of SEQ ID NO: 4 producing reduced levels of hydrogen sulfide, and comprises substitutions at position 133 and 180 that are respectively glutamic acid and aspartic acid.
62. The fermentation product of aspect 61, wherein the variant of the *SRN2* polypeptide having the amino acid sequence of SEQ ID NO: 4, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 and 180 that are respectively glutamic acid and aspartic acid.
63. The fermentation product of any one of aspects 53 and 59 to 62, wherein the polynucleotide encoding the variant of the *SRN2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 3.
64. The fermentation product of aspect 53, wherein the polynucleotide encodes the variant of the *ZRT2* polypeptide having the amino acid sequence of SEQ ID NO: 6 producing reduced levels of hydrogen sulfide, and comprises substitutions at position 19 (different from glycine), at position 322 (different from lysine), and at position 325 (different from methionine).
65. The fermentation product of aspect 64, wherein the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position 19 (different from glycine), at position 322 (different from lysine), and at position 325 (different from methionine).
66. The fermentation product of aspect 53, wherein the polynucleotide encodes the variant of the *ZRT2* polypeptide having the amino acid sequence of SEQ ID NO: 6 producing reduced levels of hydrogen sulfide, and comprises substitutions at position19, 322 and 325 that are respectively aspartic acid, glutamic acid and threonine.
67. The fermentation product of aspect 66, wherein the variant of the *ZRT2* polypeptide having the amino acid sequence of SEQ ID NO: 6, has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position 19, 322 and 325 that are respectively aspartic acid, glutamic acid and threonine.
68. The fermentation product of any one of aspects 53 and 64 to 67, wherein the polynucleotide encoding a variant of the *ZRT2* polypeptide shares at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity with SEQ ID NO: 5.
69. The fermentation product of any one of aspects 53 to 68, having reduced levels of hydrogen sulfide is produced, wherein the fermentation product is a beverage.
70. The fermentation product of any one of aspects 53 to 68, wherein the fermentation product is a grape juice must.
71. The fermentation product of any one of aspects 53 to 68, wherein the fermentation product is wine, beer, cider, or champagne.

## Claims

1. A method for producing a fermentation product having a reduced hydrogen sulfide level, the method comprising fermenting a fermentation medium with a host cell comprising:
a polynucleotide encoding a variant *YLR125W*polypeptide having at least about 70% identity to the amino acid sequence of SEQ ID NO: 2 and which comprises a substitution at position 34 (different from asparagine) and a C-terminal truncation;
a polynucleotide encoding a variant *SRN2* polypeptide having at least about 70% identity to the amino acid sequence of SEQ ID NO: 4 and which comprises substitutions at position 133 (different from glycine) and at position 180 (different from asparagine); and/or
a polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine),
wherein the fermentation product has a reduced hydrogen sulfide level.

2. A fermentation product having a reduced hydrogen sulfide level comprising a host cell comprising:
a polynucleotide encoding a variant *YLR125W*polypeptide having at least about 70% identity to the amino acid sequence of SEQ ID NO: 2 and which comprises a substitution at position 34 (different from asparagine) and a C-terminal truncation;
a polynucleotide encoding a variant *SRN2* polypeptide having at least about 70% identity to the amino acid sequence of SEQ ID NO: 4 and which comprises substitutions at position 133 (different from glycine) and at position 180 (different from asparagine); and/or
a polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine),
wherein the fermentation product has a reduced hydrogen sulfide level.

3. The method of claim 1 or the fermentation product of claim 2, wherein the host cell comprises:
said polynucleotide encoding a variant *YLR125W* polypeptide and said polynucleotide encoding a variant *SRN2* polypeptide;
said polynucleotide encoding a variant *YLR125W* polypeptide and said polynucleotide encoding a variant *ZRT2* polypeptide;
said polynucleotide encoding a variant *SRN2* polypeptide and said polynucleotide encoding a variant *ZRT2* polypeptide; or
said polynucleotide encoding a variant *YLR125W* polypeptide, said polynucleotide encoding a variant *SRN2* polypeptide, and said polynucleotide encoding a variant *ZRT2* polypeptide.

4. A method of producing an improved host cell that produces reduced levels of hydrogen sulfide, the method comprising a step of:
replacing an endogenous polynucleotide encoding a hydrogen sulfide producing *YLR125W* polypeptide with a polynucleotide encoding a variant *YLR125W* polypeptide having at least about 70% identity to the amino acid sequence of SEQ ID NO: 2 and which comprises a substitution at position 34 (different from asparagine) and a C-terminal truncation by introducing into a parent of the host cell said polynucleotide encoding the variant *YLR125W* polypeptide;
replacing an endogenous polynucleotide encoding a hydrogen sulfide producing *SRN2* polypeptide with a polynucleotide encoding a variant *SRN2* polypeptide having at least about 70% identity to the amino acid sequence of SEQ ID NO: 4 and which comprises substitutions at position 133 (different from glycine) and at position 180 (different from asparagine) by introducing into a parent of the host cell said polynucleotide encoding the variant *SRN2* polypeptide; and/or
replacing an endogenous polynucleotide encoding a hydrogen sulfide producing *ZRT2* polypeptide with a polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine) by introducing into a parent of the host cell said polynucleotide encoding the variant *ZRT2* polypeptide,
wherein the parent of the improved host cell produces hydrogen sulfide.

5. An improved host cell culture that produces reduced levels of hydrogen sulfide comprising a population of yeast cells, wherein the yeast cells comprise:
an exogenous polynucleotide encoding a variant *YLR125W* polypeptide having at least about 70% identity to the amino acid sequence of SEQ ID NO: 2 and which comprises a substitution at position 34 (different from asparagine) and a C-terminal truncation;
an exogenous polynucleotide encoding a variant *SRN2* polypeptide having at least about 70% identity to the amino acid sequence of SEQ ID NO: 4 and which comprises substitutions at position 133 (different from glycine) and at position 180 (different from asparagine); and/or
an exogenous polynucleotide encoding a variant *ZRT2* polypeptide having at least about 70% identity to the amino acid sequence of SEQ ID NO: 6 and which comprises substitutions at position 19 (different from glycine), at position 322 (different from lysine), and at position 325 (different from methionine),
wherein the improved host cell culture produces reduced hydrogen sulfide levels in comparison to a culture of parent cells.

6. The improved host cell culture of claim 5, wherein the yeast cells comprise:
said polynucleotide encoding a variant *YLR125W* polypeptide and said polynucleotide encoding a variant *SRN2* polypeptide;
said polynucleotide encoding a variant *YLR125W* polypeptide and said polynucleotide encoding a variant *ZRT2* polypeptide;
said polynucleotide encoding a variant *SRN2* polypeptide and said polynucleotide encoding a variant *ZRT2* polypeptide; or
said polynucleotide encoding a variant *YLR125W* polypeptide, said polynucleotide encoding a variant *SRN2* polypeptide, and said polynucleotide encoding a variant *ZRT2* polypeptide.

7. The method of any one of claims 1, 3 and 4, the fermentation product of claim 2 or 3, or the improved host cell culture of claim 5 or 6, wherein:
(a) the variant *YLR125W* polypeptide produces reduced levels of hydrogen sulfide, optionally wherein the variant *YLR125W* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or higher sequence identity to the amino acid sequence of SEQ ID NO: 2; or
(b) the variant *YLR125W* polypeptide produces reduced levels of hydrogen sulfide, and comprises a substitution the amino acid at position 34 of the *YLR125W* polypeptide (lysine) and a C-terminal truncation, optionally wherein the variant *YLR125W* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 2, and comprises a lysine at position 34 and a C-terminal truncation.

8. The method of any one of claims 1, 3, 4 and 7, the fermentation product of any one of claims claim 2, 3 and 7, or the improved host cell culture of any one of claims 5 to 7, wherein the polynucleotide encoding the variant *YLR125W* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to SEQ ID NO: 1.

9. The method of any one of claims 1, 3 and 4, the fermentation product of claim 2 or 3, or the improved host cell culture of claim 5 or 6, wherein:
(a) the variant *SRN2* polypeptide produces reduced levels of hydrogen sulfide, optionally wherein the variant *SRN2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 (different from glycine) and at position 180 (different from asparagine); or
(b) the variant *SRN2* polypeptide produces reduced levels of hydrogen sulfide, and comprises substitutions at position 133 and 180 that are respectively glutamic acid and aspartic acid, optionally wherein the variant *SRN2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 4, and comprises substitutions at position 133 and 180 that are respectively glutamic acid and aspartic acid.

10. The method of any one of claims 1, 3, 4 and 9, the fermentation product of any one of claims claim 2, 3 and 9 or the improved host cell culture of any one of claims 5, 6 and 9, wherein the polynucleotide encoding the variant *SRN2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to SEQ ID NO: 3.

11. The method of any one of claims 1, 3 and 4, the fermentation product of claim 2 or 3, or the improved host cell culture of claim 5 or 6, wherein:
(a) the variant *ZRT2* produces reduced levels of hydrogen sulfide, optionally wherein the variant *ZRT2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprises substitutions at position 19 (different from glycine), at position 322 (different from is not lysine) and at position 325 (different from methionine); or
(b) the variant *ZRT2* polypeptide produces reduced levels of hydrogen sulfide, and comprises a substitution at position 19 that is aspartic acid, a substitution at position 322 that is glutamic acid and at position 325 that is threonine, optionally wherein the variant *ZRT2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to the amino acid sequence of SEQ ID NO: 6, and comprising a substitution at position 19 that is aspartic acid, a substitution at position 322 that is glutamic acid and a substitution at position 325 that is threonine.

12. The method of any one of claims 1, 3, 4 and 11, the fermentation product of any one of claims claim 2, 3 and 11 or the improved host cell culture of any one of claims 5, 6 and 11, wherein the polynucleotide encoding a variant *ZRT2* polypeptide has at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or higher sequence identity to SEQ ID NO: 5.

13. The method of any one of claims 1, 3 and 7 to 12, wherein a fermentation product having reduced levels of hydrogen sulfide is produced, and wherein:
(a) the fermentation product is a beverage;
(b) the fermentation product is a wine, a beer, a cider or a champagne;
(c) the fermentation medium is a juice, a must or a wort;
(d) the fermentation product is a grape juice must; or
(e) the fermentation product is a grape juice must and the fermentation medium is a juice or a must.

14. The fermentation product of any one of claims 2, 3 and 7 to 12, wherein:
(a) the fermentation product is a beverage;
(b) the fermentation product is a grape juice must; or
(c) the fermentation product is a wine, a beer, a cider or a champagne.
